# EUROPEAN PATENT APPLICATION

(11) **EP 4 245 364 A1**
(43) Date of publication of application: **20.09.2023**
(21) Application number: 21904836.0
(22) Date of filing: 24.03.2021
(51) Int. Cl.: A61N 7/00

(54) **FIVE-DEGREE-OF-FREEDOM THERAPEUTIC FOCUS POSITIONING DEVICE FOR MAGNETIC RESONANCE GUIDANCE**

(30) Priority: 17.12.2020 CN 202023048152 U
(71) Applicant: Shanghai Shende Green Medical Era Healthcare Technology Co., Ltd., Shanghai 200233 (CN); Shende (Ningbo) Medical Device Technology Co., Ltd, Ningbo, Zhejiang 315832 (CN); Nantong Shende Medical Device Technology Co., Ltd, Nantong, Jiangsu 226014 (CN)
(72) Inventor: SU, Zhiqiang, Shanghai 200233 (CN); TIAN, Zhou, Shanghai 200233 (CN); LIU, Zhi, Shanghai 200233 (CN); WANG, Jian, Shanghai 200233 (CN); SUN, Qi, Shanghai 200233 (CN); LIU, Wenjie, Shanghai 200233 (CN); WEN, Jiabao, Shanghai 200233 (CN); CUI, Lei, Shanghai 200233 (CN); WU, Hao, Shanghai 200233 (CN); ZHANG, Shengfa, Shanghai 200233 (CN)
(74) Representative: Savi, Massimiliano
(86) International application number: PCT/CN2021/082584
(87) International publication number: WO 2022/126896

(57) **Abstract**

Disclosed is a five-degree-of-freedom (5-DOF) therapeutic focus positioning device for magnetic resonance guidance, including: a driving bed body, a working bed body and an auxiliary bed body; the working bed body is provided with a probe connection plate and a probe movement control assembly for controlling the probe connection plate to perform a multi-degree-of-freedom movement, and the probe connection plate is provided with an ultrasonic probe; the probe movement control assembly includes a mechanism moving front and rear of the probe, a mechanism moving left and right of the probe, a rotation mechanism of the probe, a mechanism moving up and down of the probe and a swinging mechanism of the probe; and the driving bed body is provided with a motor variable speed group for driving the probe movement control assembly, the motor variable speed group provides a power for the probe movement control assembly, and controls a movement of the probe connection plate through coupling, so as to control a movement of the ultrasonic probe. The present application adopts the modular design of the driving bed, the working bed body and the auxiliary bed, and completely separates the motor variable speed group from the ultrasonic probe, which effectively avoids the electromagnetic interference caused by the close distance between the motor group and the ultrasonic probe during the operation of the device. The parallel structure design of five degrees of freedom makes the movement process precisely controlled, and the degrees of freedom do not interfere with each other. The positioning of the probe focus is more flexible and convenient.

## Description

### TECHNICAL FIELD

The present application relates to the technical field of medical device, and in particular to a five-degree-of-freedom (5-DOF) therapeutic focus positioning device for magnetic resonance guidance (MRI-guided).

### BACKGROUND

The MR-guided focused ultrasound surgery (MrgFUS) is a non-invasive non-invasive therapy technique based on real-time magnetic resonance imaging. In recent years, it has shown good results in the clinical therapy of uterine fibroids, brain tumors and other tumors. With the continuous development of real-time imaging technology, this physical therapy technology can be monitored in real time during the therapy process, which greatly improves the accuracy of therapy, and has attracted more and more attention from the medical community.

When the phased-array high-intensity focused ultrasound therapy system works under the guidance of imaging equipment such as magnetic resonance, it needs to obtain images around the diseased tissue in real time from the equipment, which is convenient for doctors to plan therapy before the surgery and to evaluate the therapy effect after the surgery. The focus of the phased-array high-intensity focused ultrasound therapy system is the movable ultrasound probe. When the doctor determines the ablation area and prepares for the ablation operation, the accurate and reliable positioning of the ultrasound probe plays a key role in selectively ablation of the diseased tissue. The positioning of the ultrasound probe requires precise mechanical controlled device. Therefore, whether an accurate and reliable probe mechanical controlled device can be invented directly determines the length of the therapy time and the accuracy of the therapy range of the lesion, which has a direct impact on the safety of the therapy process and the effect after the therapy.

As a result, various probe controlled equipment came into being. CN108042932A discloses an ultrasonic probe controlled device used in the Magnetic Resonance Imaging Phased High Intensity Focused Ultrasound (MRI-pHIFU) hyperthermia system, and discloses a probe movement control assembly for controlling the probe connection plate to perform the multi-degree-of-freedom movement. A motor variable speed group for driving the probe movement control mechanism is provided in the driving bed, and the probe movement control assembly includes a probe front and rear movement mechanism, a probe left and right movement mechanism, a probe rotation mechanism and a probe swing mechanism. The probe connection plate is installed with an ultrasonic probe. However, the motor used in the mechanical positioning device will affect the performance of the magnetic resonance, or the motor is away from the magnetic resonance, resulting in a relatively great movement accuracy error.

### SUMMARY

The present application provides a 5-DOF therapy focus positioning device for magnetic resonance guidance in order to solve the above problems.

The purpose of the application is realized by the following technical solutions.

A 5-DOF therapeutic focus positioning device for magnetic resonance guidance, including: a driving bed body, a working bed body and an auxiliary bed body;
the working bed body is provided with a probe connection plate and a probe movement control assembly for controlling the probe connection plate to perform a multi-degree-of-freedom movement, and the probe connection plate is provided with an ultrasonic probe;
the probe movement control assembly includes a mechanism moving front and rear of the probe, a mechanism moving left and right of the probe, a rotation mechanism of the probe, a mechanism moving up and down of the probe and a swinging mechanism of the probe; and
the driving bed body is provided with a motor variable speed group for driving the probe movement control assembly, the motor variable speed group provides a power for the probe movement control assembly, and controls a movement of the probe connection plate through coupling, so as to control a movement of the ultrasonic probe.

The present application adopts the modular design of the driving bed, the working bed body and the auxiliary bed, and completely separates the motor variable speed group from the ultrasonic probe, which effectively avoids the electromagnetic interference caused by the close distance between the motor group and the ultrasonic probe during the operation of the device. The parallel structure design of five degrees of freedom makes the movement process precisely controlled, and the degrees of freedom do not interfere with each other. The positioning of the probe focus is more flexible and convenient.

In an embodiment, five motor variable speed groups are provided, including a motor variable speed group of the mechanism moving front and rear of the probe, a motor variable speed group of the mechanism moving left and right of the probe, a motor variable speed group of the rotation mechanism of the probe, a motor variable speed group of the rotation mechanism of the probe and a motor variable speed group of the swing mechanism of the probe, and
the motor variable speed group of the mechanism moving front and rear of the probe is fixed on the driving bed body, the motor variable speed group of the mechanism moving left and right of the probe is fixed on a dragging plate of a driving area through a first triangular bracket, the motor variable speed group of the rotation mechanism of the probe is fixed on the dragging plate of the driving area through a second triangular bracket, the motor variable speed group of the rotation mechanism of the probe is fixed on the dragging plate of the driving area through a third triangular bracket, the motor variable speed group of the swing mechanism of the probe is fixed on the dragging plate of the driving area through a fourth triangular bracket, and the dragging plate of the driving area is coupled to the driving bed body through a guiding rail of the driving area.

In an embodiment, each motor variable speed group includes a motor frame, a first gear and a second gear provided in the motor frame, the first gear is larger than the second gear, the second gear is meshed with the first gear, an ultrasonic motor is fixed on the second gear, the first gear is fixed to an output shaft of the motor, and the output shaft of the motor is coupled on the motor frame through a bearing of the motor group.

In an embodiment, the mechanism moving front and rear of the probe is embedded on a dovetail groove shape guiding rail through a sliding rail, the dovetail groove shape guiding rail is coupled on the working bed body through a fixing bracket of the guiding rail, and a dragging plate moving left and right of the working area is connected to the motor variable speed group of the mechanism moving front and rear of the probe and move front and rear through a front and rear connection assembly.

In an embodiment, the front and rear connection assembly includes a rod pushing front and rear and a screw rod moving front and rear, one end of the rod pushing left and right is connected to the dragging plate moving left and right of the working area through a supporting member moving front and rear, another end of the rod pushing front and rear is fixed to the dragging plate of the driving area through an adjustment block, one end of the screw rod moving front and rear is connected to the motor variable speed group of the mechanism moving front and rear of the probe through a coupling, and another end of the screw rod moving front and rear is coupled to a nut of the screw rod moving front and rear.

In an embodiment, the mechanism moving left and right of the probe includes a dragging plate of the working area coupled on the dragging plate moving left and right of the working area through a sliding rail moving left and right, the dragging plate of the working area is connected to a first transmission unit, configured to change a direction of a power, through a transmission screw rod moving left and right, the first transmission unit is connected to the motor variable speed group of the mechanism moving left and right of the probe and move left and right through a dragging plate moving left and right of the working area and a coupling.

In an embodiment, the first transmission unit includes a first bevel gear and a second bevel gear meshed with the first bevel gear, the first bevel gear is fixed to an end of the dragging plate moving left and right of the working area, the second bevel gear is fixed on an end of the transmission screw rod moving left and right, the dragging plate moving left and right of the working area is provided with a first bearing of the working area and is supported by a first bearing bracket, a first bearing bracket, a square bearing cover is provided outside the first bearing of the working area, the transmission screw rod moving left and right is provided with a second bearing of the working area and is supported by a second bearing bracket, a circular bearing cover is provided outside the second bearing of the working area, and the transmission screw rod moving left and right is fixed on the dragging plate of the working area through a screw rod bracket.

In an embodiment, the swinging mechanism of the probe includes a transmission worm screw rod, a transmission worm rod connected to the transmission worm screw rod, a transmission worm rod meshed with the transmission worm rod, and a rotation sleeve connected to the transmission worm rod, the rotation sleeve is fixed to a rotation frame, a rotation movement is generated between the rotation frame and a first rotation side plate, a second rotation side plate is configured to generate a rotation movement, one end of the transmission worm screw rod is provided on a worm rod bracket, another end of the transmission worm screw rod is connected to a second transmission unit configured to change the direction of the power, the second transmission unit is connected to the motor variable speed group of the rotation mechanism of the probe through a second coupling of the driving area and a rotation shaft transmitting front and rear, the transmission worm rod is provided in the worm rod bracket, and the dragging plate of the working area is provided with a sleeve limiter confining a front and rear movement of the rotation sleeve.

In an embodiment, the second transmission unit includes a third bearing of the working area, a fourth bearing of the working area, a third bearing bracket, a fourth bearing bracket, a second circular bearing cover, a second square bearing cover, a third bevel gear and a fourth bevel gear, the rotation shaft transmitting front and rear is coupled to the third bearing bracket through the third bearing of the working area and is fixed to the third bevel gear, the second circular bearing cover is clamped in the third bearing of the working area and is fixed on the third bearing bracket, the third bevel gear is meshed with the fourth bevel gear, the fourth bevel gear is fixed to the transmission worm screw rod, the transmission worm screw rod is coupled with the fourth bearing bracket through the fourth bearing of the working area, and the second square bearing cover is clamped on the fourth bearing of the working area and is fixed on the fourth bearing bracket.

In an embodiment, the rotation mechanism of the probe includes a motor variable speed group of the rotation mechanism of the probe, a nut of the rotation mechanism, a nut fixing frame of the rotation mechanism, a rotation screw rod, a rotation guiding rod, a rotation shaft, a rotation crank, the rotation crank, a rotation transmission shaft, a rotation block, a first connecting rod, and a second connecting rod, the motor variable speed group of the rotation mechanism of the probe realizes a front and rear movement of the rotation screw rod through rotating the nut of the rotation mechanism, the rotation screw rod realizes a front and rear movement of a rotation shaft through rotating the rotation guiding rod, there is no limitation when the rotation guiding rod and the rotation shaft move left and right, a motion is transmitted to the rotation block by the rotation shaft through the rotation crank and the rotation transmission shaft, the probe connection plate is directly driven to rotate by the rotation block through the first connecting rod and the second connecting rod, wherein the rotation shaft is rotably coupled with the rotation crank, the rotation crank is fixed to the rotation transmission shaft, and the rotation transmission shaft is fixed to the rotation block.

In an embodiment, The mechanism moving up and down of the probe includes the motor variable speed group of the mechanism moving up and down of the probe, a nut of the up and down movement mechanism, a nut bracket of the up and down movement mechanism, a screw rod of the up and down movement mechanism, a guiding rod of the up and down movement mechanism, a transmission shaft of the up and down movement mechanism, a conduction block of the up and down movement mechanism, a crank of the up and down movement mechanism, a connecting block of the up and down movement mechanism, a supporting bracket of the probe of the up and down movement mechanism, and the probe connection plate. The motor variable speed group of the mechanism moving up and down of the probe converts the rotation movement to a liner movement of the screw rod of the up and down movement mechanism through the nut of the up and down movement mechanism. The screw rod of the up and down movement mechanism passes the movement to the up and down movement mechanism through the guiding rod of the up and down movement mechanism, and the conduction block of the up and down movement mechanism is driven to move. The connecting block of the up and down movement mechanism is directly driven to move by the conduction block of the up and down movement mechanism through the crank of the up and down movement mechanism. The connecting block of the up and down movement mechanism is fixed to the supporting bracket of the probe of the up and down movement mechanism. The supporting bracket of the probe of the up and down movement mechanism is fixed to the probe connection plate, and the probe is driven to rotate up and down, so as to realize the up and down movement of the probe. There is no limitation when the screw rod of the up and down movement mechanism and the guiding rod of the up and down movement mechanism move left and right.

The whole machine of the present application is non-magnetic and fully compatible with the MRI equipment. The feasibility of the movement and the performance have been verified in the experiment of ablation of bone tumors in the MRI-pHIFU hyperthermia system. The machine in the present application can assist the ultrasonic system to accurately move and position the focal point during the therapy process to make up for the lack of spatial movement range of the phased-array focus, which is of great significance for realizing ablation of large-scale bone tumors, reducing ablation errors, and realizing the precise therapy.

Compared with prior art, the present application has the following advantages:
1. The present application adopts the modular design of the driving bed, the working bed body and the auxiliary bed, and completely separates the motor variable speed group from the ultrasonic probe, which effectively avoids the electromagnetic interference caused by the close distance between the motor group and the ultrasonic probe during the operation of the device. However, CN108042932A only provides two modules of the driving bed body and the working bed. In view of the movement range of the probe, the present application increases the degree of freedom in the up and down direction, greatly improving the range of the therapy. Specifically, the motor variable speed group of the mechanism moving up and down of the probe drives the nut of the mechanism moving up and down, the nut bracket of the mechanism moving up and down, the screw rod of the mechanism moving up and down, the guiding rod of the mechanism moving up and down, the transmission shaft of the mechanism moving up and down, the conduction block of the mechanism moving up and down, the crank of the mechanism moving up and down, the connecting block of the mechanism moving up and down, and the probe supporting frame of the mechanism moving up and down enable the probe connecting plate to rotate within a specified angle range up and down, and the movement space is greatly improved.
2. The parallel structure design of five degrees of freedom makes the movement process precisely controlled, and the degrees of freedom do not interfere with each other. The positioning of the probe focus is more flexible and convenient, and more optional angles and directions are provided for the therapy of different parts of the human body. CN108042932A is a four-axis mechanism, the position and posture that the probe can reach are limited, and the ideal position and posture of the probe will not be realized in the therapy process of some tumors. Specifically, the guiding rod of the mechanism moving up and down transmits the movement to the transmission shaft of the mechanism moving up and down, the conduction block of the mechanism moving up and down is driven to move, the connecting block of the mechanism moving up and down is driven to move through the crank of the mechanism moving up and down, and the probe is driven to rotate in the up and down direction through the moving mechanism. The realization of this process increases the 360-degree free space swing and rotation of the probe, which greatly improves the therapy effect.

### BRIEF DESCRIPTION OF THE DRAWINGS

FIG. 1 and FIG. 2 are whole structural schematic views of the present application.
FIG. 3 is a structural schematic view of a motor variable speed group of the present application.
FIG. 4 is an internal structural schematic view of the motor variable speed group of the present application.
FIG. 5 is a structural schematic view of a mechanism moving front and rear of a probe of the present application.
FIG. 6 is a schematic structural view of a mechanism moving left and right of the probe of the present application.
FIG. 7 is a schematic structural view of a swinging mechanism of the probe of the present application.
FIG. 8 is a partially enlarged schematic view of FIG. 7.
FIG. 9 and FIG. 10 are structural schematic views of a rotation mechanism of the probe of the present application.
FIG. 11 and FIG. 12 are structural schematic views of a mechanism moving up and down of the probe.

### DETAILED DESCRIPTION OF THE EMBODIMENTS

The present application will be described in detail below with the accompanying drawings and embodiments.

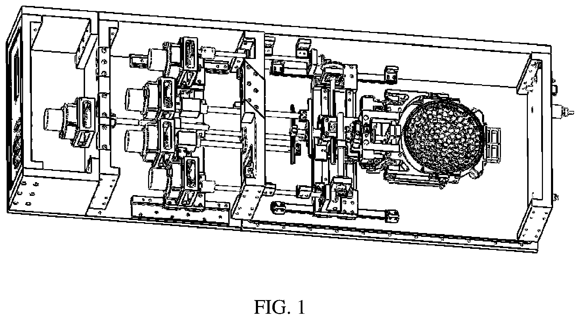

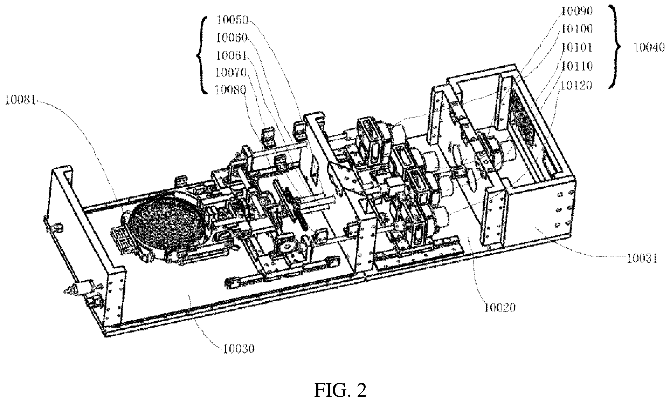

An ultrasonic probe controlled device used in a Magnetic Resonance Imaging Phased High Intensity Focused Ultrasound (MRI-pHIFU) hyperthermia system, as shown in FIG. 1 and FIG. 2, includes a driving bed body 10020, a working bed body 10030 and an auxiliary bed body 10031. A probe connection plate 10081 and a probe movement control assembly for controlling the probe connection plate 10081 to perform a multi-degree-of-freedom movement are provided in the working bed body 10030. A motor variable speed group 10040 is provided in the driving bed body 10020 for driving the probe movement control assembly. The probe movement control assembly includes a mechanism moving front and rear of the probe 10070, a mechanism moving left and right of the probe 10060, a rotation mechanism of the probe 10050, an mechanism moving up and down of the probe 10061 and a swinging mechanism of the probe 10080. The probe connection plate 10081 is equipped with an ultrasonic probe, the motor variable speed group 10040 provides a power for the probe movement control assembly, and controls a movement of the probe connection plate 10081 through coupling, thereby controlling a movement of the ultrasonic probe.

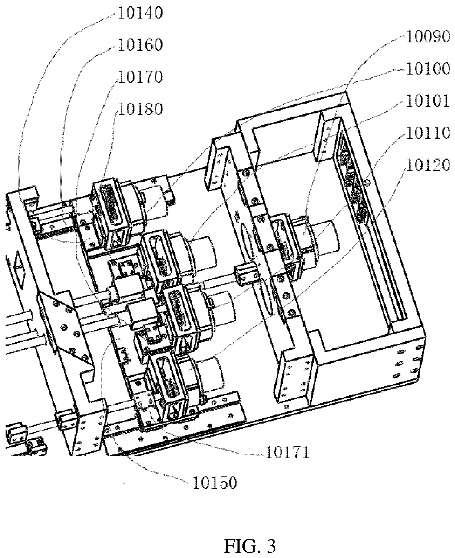

As shown in FIG. 3, five motor variable speed groups 10040 are provided, including a motor variable speed group of the mechanism moving front and rear of the probe 10090, a motor variable speed group of the mechanism moving left and right of the probe 10100, a motor variable speed group of the rotation mechanism of the probe 10101, a motor variable speed group of the mechanism moving up and down of the probe 10110 and a motor variable speed group of the swing mechanism of the probe 10120. The motor variable speed group of the mechanism moving front and rear of the probe 10090 is fixed on the driving bed body 10020, the motor variable speed group of the mechanism moving left and right of the probe 10100 is fixed on a dragging plate of a driving area 10150 though a first triangular bracket 10140, the motor variable speed group of the rotation mechanism of the probe 10101 is fixed on the dragging plate of the driving area 10150 though a second triangular bracket 10180, the motor variable speed group of the mechanism moving up and down of the probe 10110 is fixed on the dragging plate of the driving area 10150 though a third triangular bracket 10170, the motor variable speed group of the swing mechanism of the probe 10120 is fixed on the driving area 10150 though a fourth triangular bracket 10171, and the dragging plate of the driving area 10150 is coupled on the driving bed body 10020 through a guiding rail of the driving area 10160.

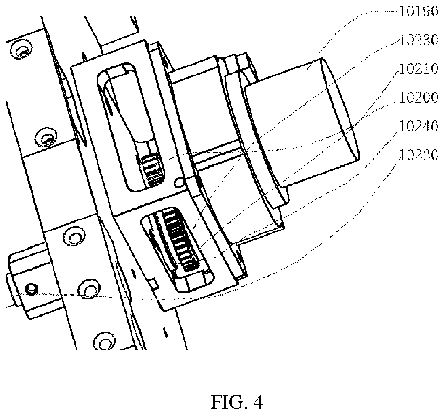

As shown in FIG. 4, each motor variable speed group includes a motor frame 10240, a first gear 10210 and a second gear 10200 installed in the motor frame 10240, the first gear 10210 is larger than the second gear 10200, and the second gear 10200 is meshed with the first gear 10210. The second gear 10200 is fixed to an ultrasonic motor 10190, the first gear 10210 is fixed with an output shaft of the motor 10220, and the output shaft of the motor 10220 is coupled to the motor frame 10240 through a bearing of the motor group 10230.

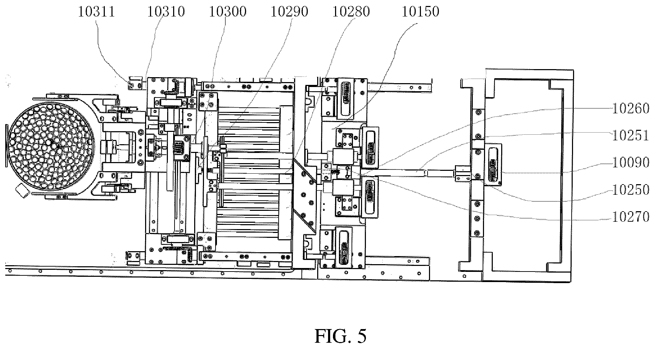

As shown in FIG. 5, the mechanism moving front and rear of the probe 10070 is embedded on a dovetail groove shape guiding rail 10310 through the sliding rail, and the dovetail groove shape guiding rail 10310 is coupled on the working bed body 10030 through a fixing bracket of the guiding rail 10311. A dragging plate moving left and right of the working area 10300 moves left and right through that a front and rear connecting assembly is connected to the motor variable speed group of the mechanism moving front and rear of the probe 10090. The front and rear connection assembly includes a rod pushing front and rear 10280 and a screw rod moving front and rear 10251. One end of the rod pushing left and right 10280 is connected to the dragging plate moving left and right of the working area through a supporting member moving front and rear 10290, another end of the rod pushing front and rear 10280 is fixed to the dragging plate of the driving area 10150 through an adjustment block 10270. One end of the screw rod moving front and rear 10251 is connected to the motor variable speed group of the mechanism moving front and rear of the probe 10090 through a coupling 10250, another end of the screw rod moving front and rear 10251 is coupled to a nut of the screw rod moving front and rear 10260 fixed on the dragging plate of the driving area 10150.

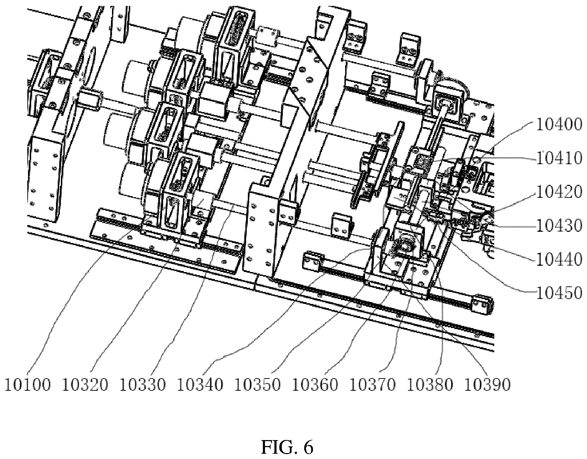

As shown in FIG. 6, the mechanism moving left and right of the probe 10060 includes a dragging plate of the working area 10440 coupled on the dragging plate moving left and right of the working area 10300 through a sliding rail moving left and right 10450. The dragging plate of the working area 10440 is connected to a first transmission unit, which can change a direction of the power, through a transmission screw rod moving left and right 10420. The first transmission unit is connected to the motor variable speed group of the mechanism moving left and right of the probe 10100 to move left and right through the dragging plate moving left and right of the working area 10300 and a coupling 10320. The first transmission unit includes a first bevel gear 10350 and a second bevel gear 10360 meshed with the first bevel gear 10350. The first bevel gear 10350 is fixed to one end of a transmission screw rod moving front and rear 10330, the second bevel gear 10360 is fixed to an end of the transmission screw rod moving left and right 10420. A first bearing of the working area 10340 is provided on the transmission screw rod moving front and rear 10330, and is supported and fixed by a first bearing bracket 10390. A square bearing cover 10410 is provided outside the first bearing of the working area 10340, a second bearing of the working area 10370 is provided on the transmission screw rod moving left and right 10420 and is supported and fixed by a second bearing bracket 10400. A circular bearing cover 10380 is provided outside the second bearing of the working area 10370, the transmission screw rod moving left and right 10420 is fixed on the dragging plate of the working area 10440 through a screw rod bracket 10430.

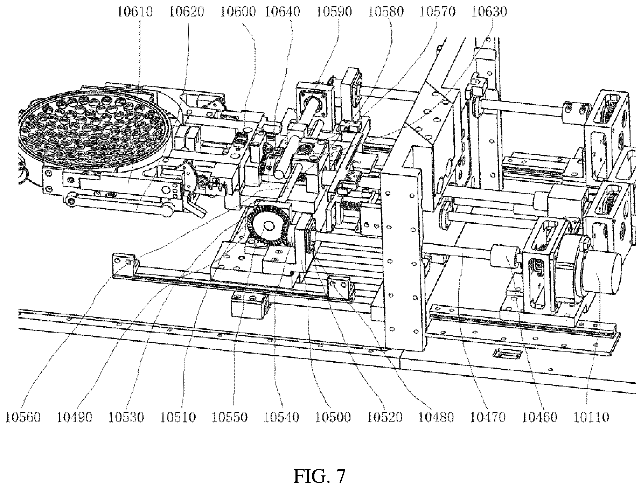

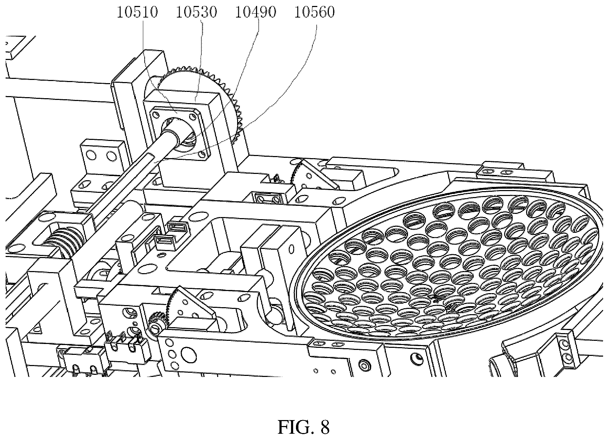

As shown in FIG. 7 to FIG. 8, the swinging mechanism of the probe 10080 includes a transmission worm screw rod 10560, a transmission worm rod 10570 connected to the transmission worm screw rod 10560, a transmission turbine 10580 meshed with the transmission worm rod 10570. A rotation sleeve 10590 is connected to the transmission worm rod 10580, and is fixed to the rotation frame 10600. A rotation movement is generated between the rotation frame 10600 and a first rotation side plate 10610, and can also be generated with the second rotation side plate 10620. One end of the transmission worm screw rod 10560 is installed on a worm rod bracket 10630, another end of the transmission worm screw rod 10560 is connected to a second transmission unit which can change the direction of the power. The second transmission unit is connected to the motor variable speed group of the mechanism moving up and down of the probe 10110 through a second coupling of the driving area 10460 and a rotation shaft transmitting front and rear 10470. The transmission worm rod 10570 is provided in the worm rod bracket 10630, the dragging plate of the working area 10440 is provided with a sleeve limiter 10640 that confines the front and rear movement of the rotation sleeve 10590. The second transmission unit includes a third bearing of the working area 10480, a fourth bearing of the working area 10490, a third bearing bracket 10500, a fourth bearing bracket 10510, a second circular bearing cover 10520, a second square bearing cover 10530, a third bevel gear 10540 and a fourth bevel gear 10550. The rotation shaft transmitting front and rear 10470 is coupled with the third bearing bracket 10500 through the third bearing of the working area 10480, and is fixed to the third bevel gear 10540. The second circular bearing cover 10520 is clamped on the third bearing of the working area 10480 and is fixed to the third bearing bracket 10500, the third bevel gear 10540 is meshed with the fourth bevel gear 10550, the fourth bevel gear 10550 is fixed to the transmission worm screw rod 10560, the transmission worm screw rod 10560 is coupled with the fourth bearing bracket 10510 through the fourth bearing of the working area 10490, and the second square bearing cover 10530 is clamped on the fourth bearing of the working area 10490 and is fixed on the fourth bearing bracket 10510.

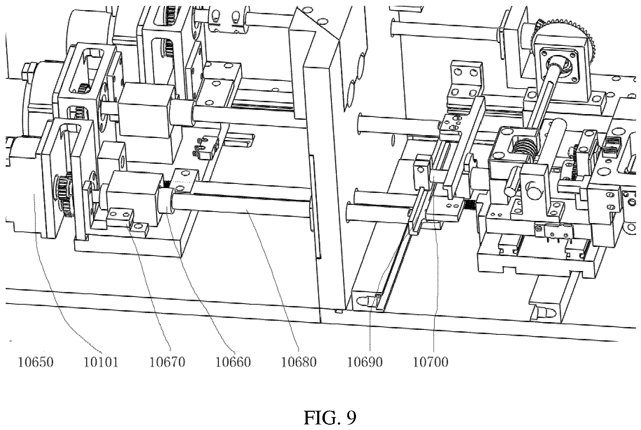

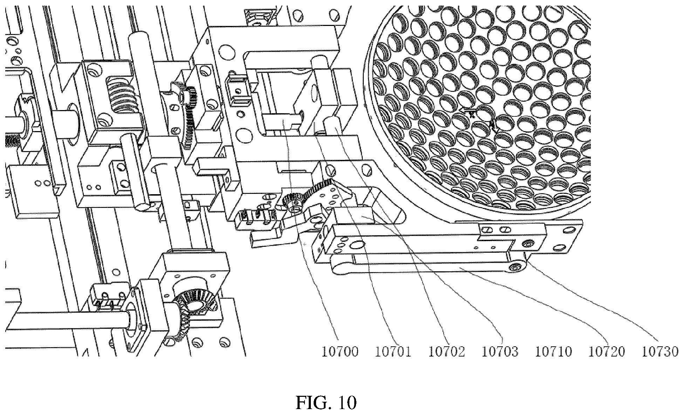

As shown in FIG. 9 to FIG. 10, the rotation mechanism of the probe 10050 includes the motor variable speed group of the rotation mechanism of the probe 10101, a nut of the rotation mechanism 10660, a nut fixing frame of the rotation mechanism 10670, a rotation screw rod 10680, a rotation guiding rod 10690, a rotation shaft 10700, a rotation crank 10701, the rotation crank 10701, a rotation transmission shaft 10702, a rotation block 10703, a first connecting rod 10720, and a second connecting rod 10730. The nut of the rotation mechanism 10660 is rotated by the motor variable speed group of the rotation mechanism of the probe 10101 so that the rotation screw rod 10680 can move front and rear, the rotation guiding rod 10690 is rotated by the rotation screw rod 10680 so that the rotation shaft 10700 can move front and rear, and there is no limitation when the rotation guiding rod 10690 and the rotation shaft 10700 move left and right. The rotation block 10703 is made to move by the rotation shaft 10700 through the rotation crank 10701 and the rotation transmission shaft 10702, and the probe connection plate 10081 is directly rotated by the rotation block 10703 through the first connecting rod 10720 and the second connecting rod 730. The rotation shaft 10700 is rotabaly coupled with the rotation crank 10701, the rotation crank 10701 is fixed with the rotation transmission shaft 10702, and the rotation transmission shaft 10702 is fixed with the rotation block 10703.

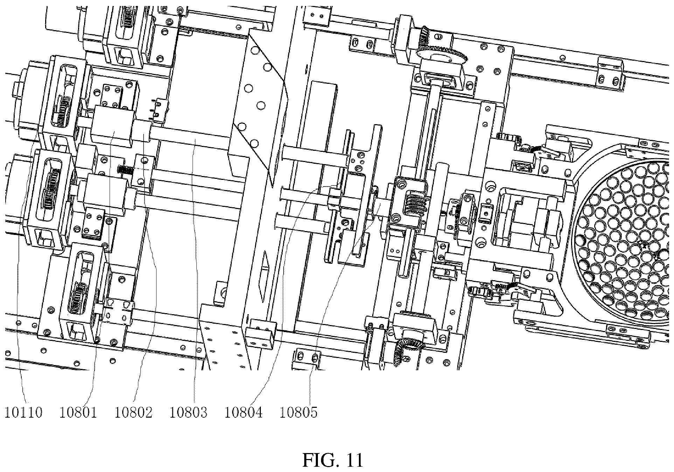

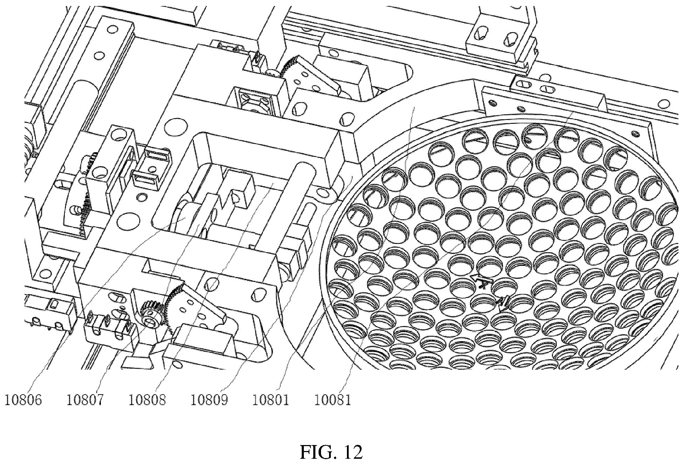

As shown in FIG. 11 and FIG. 12, the mechanism moving up and down of the probe 10061 includes the motor variable speed group of the mechanism moving up and down of the probe 10110, a nut of the up and down movement mechanism 10802, a nut bracket of the up and down movement mechanism 10801, a screw rod of the up and down movement mechanism 10803, a guiding rod of the up and down movement mechanism 10804, a transmission shaft of the up and down movement mechanism 10805, a conduction block of the up and down movement mechanism 10806, a crank of the up and down movement mechanism 10807, a connecting block of the up and down movement mechanism 10808, a supporting bracket of the probe of the up and down movement mechanism 10809, and the probe connection plate 10081. The motor variable speed group of the mechanism moving up and down of the probe 10110 converts the rotation movement to a liner movement of the screw rod of the up and down movement mechanism 10803 through the nut of the up and down movement mechanism 10802. The screw rod of the up and down movement mechanism 10803 passes the movement to the up and down movement mechanism 10805 through the guiding rod of the up and down movement mechanism 10804, and the conduction block of the up and down movement mechanism 10806 is driven to move. The connecting block of the up and down movement mechanism 10808 is directly driven to move by the conduction block of the up and down movement mechanism 10806 through the crank of the up and down movement mechanism 10807. The connecting block of the up and down movement mechanism 10808 is fixed to the supporting bracket of the probe of the up and down movement mechanism 10809. The supporting bracket of the probe of the up and down movement mechanism 10809 is fixed to the probe connection plate 10081, and the probe is driven to rotate up and down, so as to realize the up and down movement of the probe. There is no limitation when the screw rod of the up and down movement mechanism 10803 and the guiding rod of the up and down movement mechanism 10804 move left and right.

The above descriptions of the embodiments are for those skilled in the art to understand and use the present application. It is obvious that those skilled in the art can easily make various modifications to these embodiments, and apply the general principles to other embodiments without creative effort. Therefore, the present application is not limited to the above-mentioned embodiments. Improvements and modifications made by those skilled in the art according to the disclosure of the present application without departing from the scope of the present application should fall within the scope of the present application.

## Claims

1. A 5-DOF therapeutic focus positioning device for magnetic resonance guidance, **characterized by** comprising: a driving bed body (10020), a working bed body (10030) and an auxiliary bed body (10031); wherein
the working bed body (10030) is provided with a probe connection plate (10081) and a probe movement control assembly for controlling the probe connection plate (10081) to perform a multi-degree-of-freedom movement, and the probe connection plate (10081) is provided with an ultrasonic probe;
the probe movement control assembly comprises a mechanism moving front and rear of the probe (10070), a mechanism moving left and right of the probe (10060), a rotation mechanism of the probe (10050), a mechanism moving up and down of the probe (10061) and a swinging mechanism of the probe (10080); and
the driving bed body (10020) is provided with a motor variable speed group (10040) for driving the probe movement control assembly, the motor variable speed group (10040) provides a power for the probe movement control assembly, and controls a movement of the probe connection plate (10081) through coupling, so as to control a movement of the ultrasonic probe.

2. The 5-DOF therapeutic focus positioning device for magnetic resonance guidance according to claim 1, wherein:
five motor variable speed groups (10040) are provided, comprising a motor variable speed group of the mechanism moving front and rear of the probe (10090), a motor variable speed group of the mechanism moving left and right of the probe (10100), a motor variable speed group of the rotation mechanism of the probe (10101), a motor variable speed group of the rotation mechanism of the probe (10110) and a motor variable speed group of the swing mechanism of the probe (10120), and
the motor variable speed group of the mechanism moving front and rear of the probe (10090) is fixed on the driving bed body (10020), the motor variable speed group of the mechanism moving left and right of the probe (10100) is fixed on a dragging plate of a driving area (10150) through a first triangular bracket (10140), the motor variable speed group of the rotation mechanism of the probe (10101) is fixed on the dragging plate of the driving area (10150) through a second triangular bracket (10180), the motor variable speed group of the rotation mechanism of the probe (10110) is fixed on the dragging plate of the driving area (10150) through a third triangular bracket (10170), the motor variable speed group of the swing mechanism of the probe (10120) is fixed on the dragging plate of the driving area (10150) through a fourth triangular bracket (10171), and the dragging plate of the driving area (10150) is coupled to the driving bed body (10020) through a guiding rail of the driving area (10160).

3. The 5-DOF therapeutic focus positioning device for magnetic resonance guidance according to claim 2, wherein each motor variable speed group comprises a motor frame (10240), a first gear (10210) and a second gear (10200) provided in the motor frame (10240), the first gear (10210) is larger than the second gear (10200), the second gear (10200) is meshed with the first gear (10210), an ultrasonic motor (10190) is fixed on the second gear (10200), the first gear (10210) is fixed to an output shaft of the motor (10220), and the output shaft of the motor (10220) is coupled on the motor frame (10240) through a bearing of the motor group (10230).

4. The 5-DOF therapeutic focus positioning device for magnetic resonance guidance according to claim 2, wherein the mechanism moving front and rear of the probe (10070) is embedded on a dovetail groove shape guiding rail (10310) through a sliding rail, the dovetail groove shape guiding rail (10310) is coupled on the working bed body (10030) through a fixing bracket of the guiding rail (10311), and a dragging plate moving left and right of the working area (10300) is connected to the motor variable speed group of the mechanism moving front and rear of the probe (10090) and move front and rear through a front and rear connection assembly.

5. The 5-DOF therapeutic focus positioning device for magnetic resonance guidance according to claim 4, wherein the front and rear connection assembly comprises a rod pushing front and rear (10280) and a screw rod moving front and rear (10251), one end of the rod pushing front and rear (10280) is connected to the dragging plate moving left and right of the working area (10300) through a supporting member moving front and rear (10290), another end of the rod pushing front and rear (10280) is fixed to the dragging plate of the driving area (10150) through an adjustment block (10270), one end of the screw rod moving front and rear (10251) is connected to the motor variable speed group of the mechanism moving front and rear of the probe (10090) through a coupling (10250), and another end of the screw rod moving front and rear (10251) is coupled to a nut of the screw rod moving front and rear (10260).

6. The 5-DOF therapeutic focus positioning device for magnetic resonance guidance according to claim 2, wherein the mechanism moving left and right of the probe (10060) comprises a dragging plate of the working area (10440) coupled on the dragging plate moving left and right of the working area (10300) through a sliding rail moving left and right (10450), the dragging plate of the working area (10440) is connected to a first transmission unit, configured to change a direction of a power, through a transmission screw rod moving left and right (10420), the first transmission unit is connected to the motor variable speed group of the mechanism moving left and right of the probe (10100) and move left and right through a dragging plate moving left and right of the working area (10330) and a coupling (10320).

7. The 5-DOF therapeutic focus positioning device for magnetic resonance guidance according to claim 6, wherein the first transmission unit comprises a first bevel gear (10350) and a second bevel gear (10360) meshed with the first bevel gear (10350), the first bevel gear (10350) is fixed to an end of the dragging plate moving left and right of the working area (10330), the second bevel gear (10360) is fixed on an end of the transmission screw rod moving left and right (10420), the dragging plate moving left and right of the working area (10330) is provided with a first bearing of the working area (10340) and is supported by a first bearing bracket (10390), a first bearing bracket (10390), a square bearing cover (10410) is provided outside the first bearing of the working area (10340), the transmission screw rod moving left and right (10420) is provided with a second bearing of the working area (10370) and is supported by a second bearing bracket (10400), a circular bearing cover (10380) is provided outside the second bearing of the working area (10370), and the transmission screw rod moving left and right (10420) is fixed on the dragging plate of the working area (10440) through a screw rod bracket (10430).

8. The 5-DOF therapeutic focus positioning device for magnetic resonance guidance according to claim 2, wherein the swinging mechanism of the probe (10080) comprises a transmission worm screw rod (10560), a transmission worm rod (10570) connected to the transmission worm screw rod (10560), a transmission worm rod (10580) meshed with the transmission worm rod (10570), and a rotation sleeve (10590) connected to the transmission worm rod (10580), the rotation sleeve (10590) is fixed to a rotation frame (10600), a rotation movement is generated between the rotation frame (10600) and a first rotation side plate (10610), a second rotation side plate (10620) is configured to generate a rotation movement, one end of the transmission worm screw rod (10560) is provided on a worm rod bracket (10630), another end of the transmission worm screw rod (10560) is connected to a second transmission unit configured to change the direction of the power, the second transmission unit is connected to the motor variable speed group of the rotation mechanism of the probe (10110) through a second coupling of the driving area (10460) and a rotation shaft transmitting front and rear (10470), the transmission worm rod (10570) is provided in the worm rod bracket (10630), and the dragging plate of the working area (10440) is provided with a sleeve limiter (10640) confining a front and rear movement of the rotation sleeve (10590).

9. The 5-DOF therapeutic focus positioning device for magnetic resonance guidance according to claim 8, wherein the second transmission unit comprises a third bearing of the working area (10480), a fourth bearing of the working area (10490), a third bearing bracket (10500), a fourth bearing bracket (10510), a second circular bearing cover (10520), a second square bearing cover (10530), a third bevel gear (10540) and a fourth bevel gear (10550), the rotation shaft transmitting front and rear (10470) is coupled to the third bearing bracket (10500) through the third bearing of the working area (10480) and is fixed to the third bevel gear (10540), the second circular bearing cover (10520) is clamped in the third bearing of the working area (10480) and is fixed on the third bearing bracket (10500), the third bevel gear (10540) is meshed with the fourth bevel gear (10550), the fourth bevel gear (10550) is fixed to the transmission worm screw rod (10560), the transmission worm screw rod (10560) is coupled with the fourth bearing bracket (10510) through the fourth bearing of the working area (10490), and the second square bearing cover (10530) is clamped on the fourth bearing of the working area (10490) and is fixed on the fourth bearing bracket (10510).

10. The 5-DOF therapeutic focus positioning device for magnetic resonance guidance according to claim 4, wherein the rotation mechanism of the probe (10050) comprises a motor variable speed group of the rotation mechanism of the probe (10101), a nut of the rotation mechanism (10660), a nut fixing frame of the rotation mechanism (10670), a rotation screw rod (10680), a rotation guiding rod (10690), a rotation shaft (10070), a rotation crank (10701), the rotation crank (10701), a rotation transmission shaft (10702), a rotation block (10703), a first connecting rod (10720), and a second connecting rod (10730), the motor variable speed group of the rotation mechanism of the probe (10101) realizes a front and rear movement of the rotation screw rod (10680) through rotating the nut of the rotation mechanism (10660), the rotation screw rod (10680) realizes a front and rear movement of a rotation shaft (10700) through rotating the rotation guiding rod (10690), there is no limitation when the rotation guiding rod (10690) and the rotation shaft (10700) move left and right, a motion is transmitted to the rotation block (10703) by the rotation shaft (10700) through the rotation crank (10701) and the rotation transmission shaft (10702), the probe connection plate (10081) is directly driven to rotate by the rotation block (10703) through the first connecting rod (10720) and the second connecting rod (10730), wherein the rotation shaft (10700) is rotably coupled with the rotation crank (10701), the rotation crank (10701) is fixed to the rotation transmission shaft (10702), and the rotation transmission shaft (10702) is fixed to the rotation block (10703).
